Europäisches Patentamt

European Patent Office

Office européen des brevets

(19)

(11) Numéro de publication : **0 512 891 A1**

## (12) DEMANDE DE BREVET EUROPEEN

(21) Numéro de dépôt : **92401210.7**

(51) Int. Cl.⁵ : **G01N 33/20,** G01N 21/35, G01N 31/12

(22) Date de dépôt : **28.04.92**

(30) Priorité : **10.05.91 FR 9105697**

(43) Date de publication de la demande :
**11.11.92 Bulletin 92/46**

(84) Etats contractants désignés :
**AT BE CH DE DK ES FR GB GR IT LI LU NL PT
SE**

(71) Demandeur : **SOLLAC**
**Immeuble Elysées La Défense, 29 Le Parvis**
**F-92800 PUTEAUX (FR)**

(72) Inventeur : **Cottel, André**
**5 Chemin d'Argenlieu**
**F-60600 Lamecourt (FR)**

(74) Mandataire : **Obolensky, Michel et al**
**c/o CABINET LAVOIX 2, place d'Estienne**
**d'Orves**
**F-75441 Paris Cédex 09 (FR)**

(54) **Dispositif de mesure de la quantité d'un constituant contenu dans un échantillon de produit brûlé dans un four.**

(57) Ce dispositif comporte un analyseur à infrarouges du constituant à mesurer contenu dans l'atmosphère du four mise en circulation par une pompe dont le débit est mesuré par un débitmètre et des moyens d'intégration de la mesure instantanée de la quantité de gaz contenant le constituant, comprenant un convertisseur tension-fréquence (15) de conversion de la tension instantanée de sortie de l'analyseur en une fréquence proportionnelle à cette tension, un compteur binaire (16) des pics des signaux de fréquence émis par le convertisseur (15), un convertisseur numérique-analogique (17) des signaux de sortie du compteur (16) et délivrant un signal proportionnel à la surface délimitée par la courbe d'évolution de la teneur en gaz à mesurer et des moyens (18,19) d'affichage et d'enregistrement de la quantité de produit à mesurer contenu dans l'échantillon.

FIG.3

EP 0 512 891 A1

La présente invention est relative à l'analyse de la teneur notamment en carbone d'un échantillon de tôle et se rapporte plus particulièrement à un analyseur de mesure de la teneur en carbone de surface d'un échantillon de tôle, destiné à être associé à un four dans lequel on fait brûler l'échantillon.

On connaît des analyseurs du type à combustion comprenant un four à chambre de combustion destiné à recevoir un spécimen non gazeux, la chambre comprenant des organes d'entrée et de sortie de gaz ainsi que des organes de mise en circulation de gaz en circuit fermé dans la chambre et un détecteur relié aux organes de mise en circulation des gaz pour mesurer la concentration d'au moins un gaz prédéterminé.

On connaît également des analyseurs à combustion fonctionnant en circuit ouvert et comprenant à la sortie d'un four de combustion d'un échantillon, une pompe de mise en circulation des gaz de combustion du four, un débitmètre destiné à mesurer le débit de la pompe et un analyseur à infrarouges traversé par des gaz de combustion du four et destiné à donner une mesure de la quantité par exemple de carbone dans les gaz de combustion.

Les analyseurs connus donnent généralement une mesure instantanée de la concentration en gaz.

Or, au cours de la combustion d'un échantillon, la concentration en gaz à mesurer évolue lentement en passant par un maximum au moment où la combustion est la plus intense, puis en redescendant vers zéro.

Par conséquent, pour obtenir une mesure de la quantité totale notamment de CO2 et donc de C contenue dans un échantillon, il est nécessaire de procéder à l'intégration de la mesure instantanée, pendant la durée de combustion.

L'invention vise donc à créer un dispositif de mesure de la quantité d'un constituant contenu dans un produit qui tout en étant d'une construction relativement simple assure une mesure précise de la quantité de constituant au cours de l'évolution de la combustion du produit.

Elle a donc pour objet un dispositif de mesure de la quantité d'un constituant contenu dans un échantillon de produit, comprenant associés à un four pour brûler l'échantillon de produit, des moyens pour mettre l'atmosphère du four en circulation à travers un débitmètre et un analyseur à infrarouges qui délivre à sa sortie un signal de mesure instantanée de la quantité de gaz contenant le constituant, contenue dans l'atmosphère du four, caractérisé en ce qu'il comporte en outre des moyens d'intégration de la mesure instantanée de la quantité de gaz contenant le constituant, comprenant un convertisseur tension-fréquence de conversion de la tension instantanée de sortie de l'analyseur en une fréquence proportionnelle à la tension instantanée et un compteur binaire des pics des signaux de fréquence émis par le convertisseur et dont le compte représente la quantité de gaz contenant le constituant ayant traversé l'analyseur à infrarouges.

L'invention sera mieux comprise à l'aide de la description qui va suivre, donnée uniquement à titre d'exemple et faite en se référant aux dessins annexés, sur lesquels :

- la Fig.1 est un schéma synoptique d'un analyseur de carbone suivant l'invention;
- la Fig.2 est une vue schématique de l'analyseur à infrarouges de l'analyseur de la Fig.1;
- la Fig.3 est un schéma synoptique du circuit de traitement du signal de sortie de l'analyseur à infrarouges de la Fig.2;
- la Fig.4 est un schéma plus détaillé du convertisseur tension-fréquence du circuit de la Fig.3;
- la Fig.5 est un schéma détaillé du convertisseur numérique-analogique du circuit de la Fig.3;
- la Fig.6 est un schéma détaillé du circuit de régulation du débit de la pompe de l'analyseur de la Fig.1;
- la Fig.7 est un schéma détaillé du circuit de compensation des variations de pression atmosphérique de l'analyseur de la Fig.1;
- la Fig.8 est un diagramme représentant une courbe d'étalonnage de l'analyseur suivant l'invention; et
- la Fig.9 est un diagramme représentant une courbe de mesure obtenue à l'aide de l'analyseur suivant l'invention.

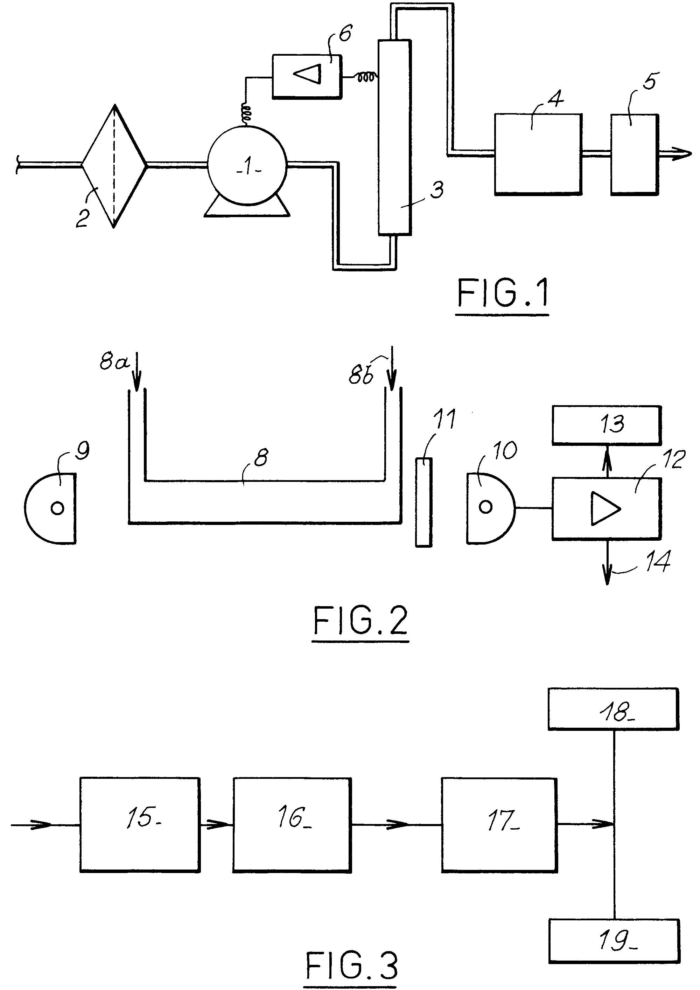

Le dispositif représenté à la Fig.1 est un analyseur de carbone qui comporte à la sortie d'un four (non représenté) destiné à faire brûler un échantillon de tôle afin de provoquer un dégagement de CO2 qui résulte de la combustion superficielle de la tôle, une pompe 1 reliée à la sortie du four par l'intermédiaire d'un filtre 2 destiné à retenir les particules de l'atmosphère gazeuse et à assécher le gaz provenant du four. La sortie de la pompe 1 est reliée à l'entrée d'un débitmètre 3 dont la sortie est à son tour reliée à un analyseur à infrarouges 4 qui sera décrit plus en détail en référence à la Fig.2 et qui est destiné à convertir la concentration en CO2 de l'atmosphère du four en un signal électrique. A la sortie de l'analyseur infrarouges 4 est disposé un circuit 5 de compensation des variations de la pression atmosphérique dont la sortie est connectée à l'entrée d'un circuit de traitement qui sera décrit en référence à la Fig.3.

L'analyseur comporte en outre un circuit 6 de régulation du débit de la pompe 1, connecté entre le débitmètre 3 et une borne de commande du moteur électrique d'entraînement de la pompe (non représenté).

L'analyseur à infrarouges représenté plus en détail à la Fig.2 comporte essentiellement une cellule 8 comprenant une entrée 8a et une sortie 8b pour le gaz à analyser. A une extrémité de la cellule réalisée en une matière transparente, est disposée une source d'infrarouges 9 tandis qu'à son extrémité opposée est placé un récepteur d'infrarouges 10, un filtre 11 spécifique au gaz à analyser, en l'occurence au CO2 étant interposé entre l'extrémité de la cellule 8 et le récepteur 10.

Bien entendu, un filtre spécifique à un autre gaz peut être substitué au filtre précité.

La sortie du récepteur à infrarouges 10 est connectée à un circuit amplificateur 12 qui à son tour est relié à un dispositif d'affichage 13 de la valeur instantanée de la concentration en CO2 du gaz de combustion du four.

L'analyseur utilisé est par exemple du type fabriqué et vendu par la société ANDROS ANALYZERS INCORPORATED, 2332 fourth street, Berkeley, CA 97710 USA. L'analyseur à infrarouges utilise l'absorption sélective par un gaz d'un faisceau de lumière infrarouge. Le filtre optique 11 disposé entre la cellule 8 et le récepteur 10 ne laisse passer que la longueur d'ondes du CO2 (nombre d'ondes du CO2 = 2361,2333 cm$^{-1}$).

Après avoir traversé le volume de la cellule, la lumière émergente est d'autant plus atténuée que la concentration en CO2 était plus forte.

Dans le présent exemple, l'analyseur délivre un signal de 1 volt linéarisé pour une concentration maximale de 1%. L'amplificateur 11 comporte de plus une sortie 14 à laquelle est connecté le circuit 5 de compensation des variations de la pression atmosphérique représenté à la Fig.1 et dont la sortie est connectée à son tour au circuit de traitement de signal représenté à la Fig.3 et qui va maintenant être décrit.

Ce circuit qui constitue en fait un intégrateur, comporte tout d'abord un convertisseur de tension-fréquence 15 qui délivre à sa sortie un signal de fréquence variable en fonction des variations de la tension qui correspond à la variation de la concentration en CO2 du gaz de combustion du four au cours de la combustion d'un échantillon. Le convertisseur tension-fréquence 15 délivre des signaux impulsionnels de fréquence variable et un compteur 16 connecté à la sortie de ce convertisseur, assure le comptage de ces impulsions. Le compteur 16 est dans le présent exemple un compteur binaire à 16 bits. La sortie du compteur 16 est reliée à un convertisseur numérique-analogique 17 de conversion du compte du compteur 16 en un signal analogique qui donne une mesure de la surface délimitée par la courbe de variation de la teneur en CO2 de l'atmosphère du four au cours de la combustion de l'échantillon dans celui-ci. A la sortie du convertisseur numérique-analogique 17 est connecté un dispositif d'affichage 18 et un dispositif d'enregistrement 19 de la valeur intégrée de la concentration en carbone au cours de toute la durée de la mesure.

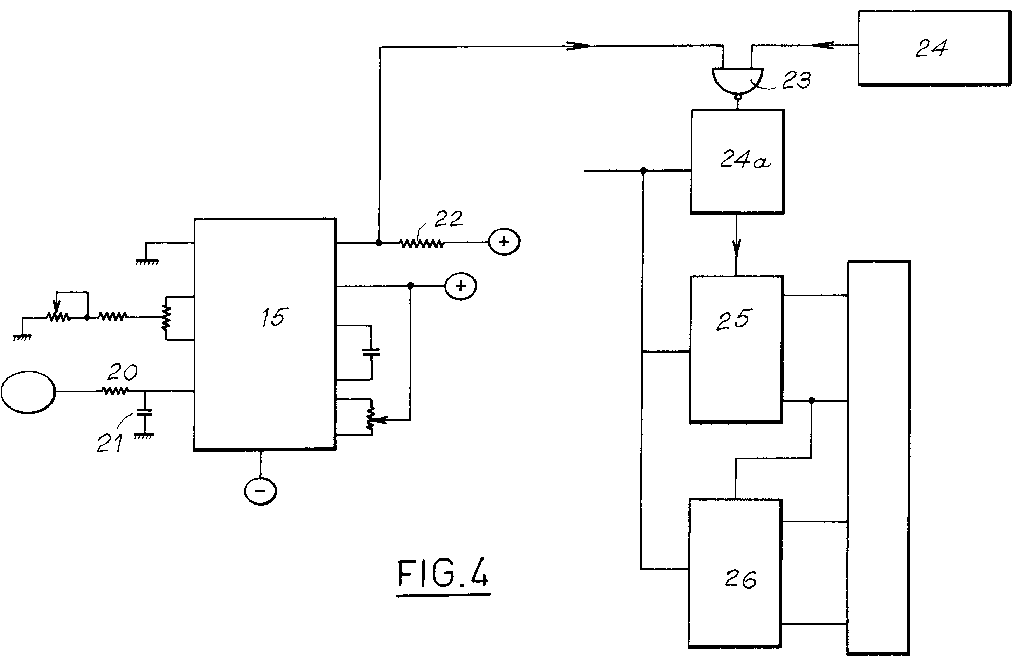

Le convertisseur tension-fréquence 15 et le compteur binaire à 16 bits 16 du circuit de la Fig.3 sont représentés plus en détail à la Fig.4.

Ce circuit comprend tout d'abord le circuit convertisseur tension-fréquence 15 qui dans le présent exemple est un circuit intégré du type AD 537 dont l'entrée analogique est connectée à la sortie du circuit 5 de compensation des variations de la pression atmosphérique de l'analyseur de la Fig.1 par l'intermédiaire d'un circuit RC 20, 21 dont le condensateur est relié entre l'entrée du convertisseur 15 et la masse.

La sortie du convertisseur 15 qui est par ailleurs connectée à une tension logique positive par l'intermédiaire d'une résistance 22 est connectée à une entrée d'une porte NON-ET 23 dont l'autre entrée est connectée à un circuit 24 de validation d'impulsions. La sortie de la porte NON-ET 23 est connectée à un diviseur par 16 24a dont la sortie est reliée à son tour à un compteur 25 à douze bits suivi d'un compteur 26 à quatre bits, les sorties parallèles des compteurs 25 et 26 constituant un mot binaire de seize bits représentant en fin de mesure la quantité de gaz carbonique ayant traversé l'analyseur au cours de cette mesure.

Les compteurs 25 et 26 sont avantageusement du type 4040 et 4024 respectivement.

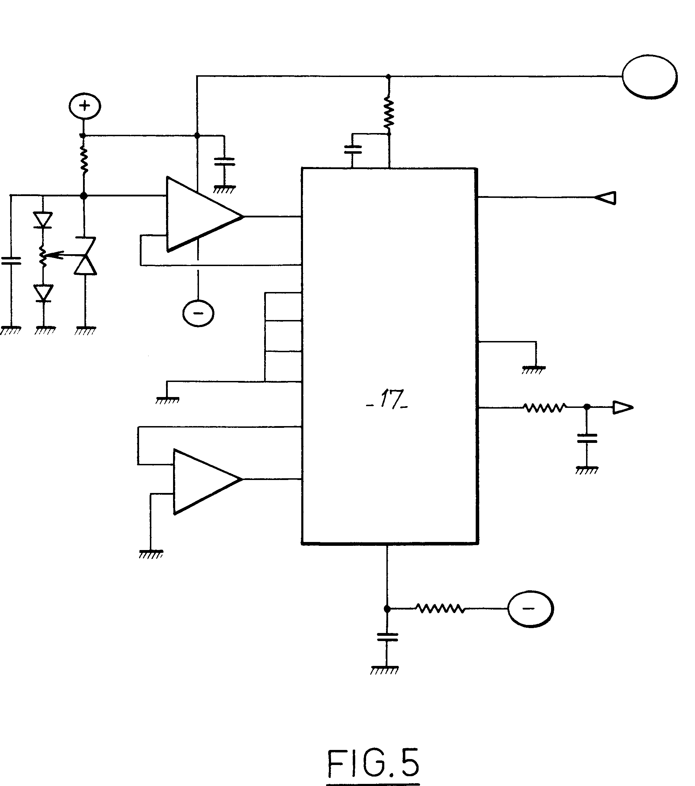

Le convertisseur numérique-analogique 17 du circuit de la Fig.3 est représenté plus en détail à la Fig.5.

Il est avantageusement du type AD 569 et reçoit sur ses entrées, les sorties D1 à D16 des compteurs 25 et 26. Il délivre à sa sortie un signal analogique qui est proportionnel au mot binaire de seize bits appliqué à ses entrées et qui correspond à la mesure de la surface délimitée par la courbe de variation de la teneur en CO2 au cours de la combustion de l'échantillon. La sortie du convertisseur 17 est connectée, comme représenté à la Fig.3, à un afficheur 18 et à un enregistreur 19.

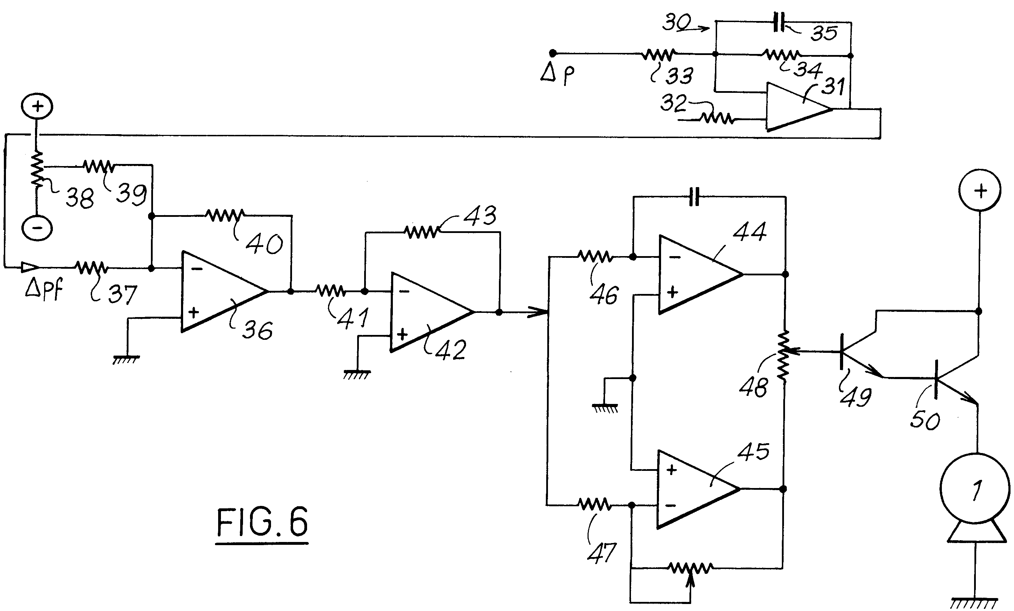

Le circuit de régulation du débit de la pompe 1 va maintenant être décrit en référence à la Fig.6.

Ce circuit, qui, comme représenté à la Fig.1 est connecté entre le débitmètre 3 et une borne de commande du moteur d'entraînement de la pompe 1 (non représenté) comporte principalement un circuit 30 de lissage du signal de sortie du débitmètre, destiné à compenser les ondulations de débit dues aux pulsations de sortie de la pompe 1. Ce circuit 30 dont l'entrée est connectée à la sortie du débitmètre 3 qui délivre un signal Δp de différence de pression entre son entrée et sa sortie, comporte essentiellement un amplificateur opérationnel 31 dont l'entrée non inverseuse est connectée à la masse par l'intermédiaire d'une résistance 32 et dont l'entrée inverseuse est connectée à la sortie du débitmètre par l'intermédiaire d'une résistance 33, cette entrée inverseuse étant reliée à la sortie de l'amplificateur par l'intermédiaire d'une résistance 34 et d'un condensateur 35 montés en parallèle.

La sortie de ce circuit de lissage est connectée à l'entrée d'un circuit de réglage de la référence de débit qui comprend un premier amplificateur monté en sommateur 36 dont l'entrée inverseuse est reliée à la sortie de l'amplificateur 31 par l'intermédiaire d'une résistance 37, un potentiomètre 38 dont le curseur est connecté par l'intermédiaire d'une résistance 39 à la même entrée non inverseuse de l'amplificateur 36, assurant le réglage de la référence de débit, tandis qu'une résistance de réaction 40 est connectée entre la sortie de l'amplificateur 36 et sa borne inverseuse.

La sortie de l'amplificateur 36 est connectée par l'intermédiaire d'une résistance 41 à l'entrée inverseuse d'un autre amplificateur 42 dont l'entrée non-inverseuse est connectée à la masse et dont la sortie est rebouclée sur son entrée inverseuse par une résistance 43.

La sortie de l'amplificateur 42 est connectée à l'entrée commune d'un amplificateur intégral 44 et d'un amplificateur proportionnel 45 par l'intermédiaire de résistances respectives 46 et 47, les sorties des amplificateurs 44 et 45 étant connectées aux bornes d'un potentiomètre 48 dont le curseur est relié à la base d'un transistor 49 de commande d'un transistor de puissance 50 dont le trajet collecteur-émetteur est connecté en série dans le circuit d'alimentation du moteur d'entraînement de la pompe 1.

Les amplificateurs opérationnels utilisés dans ce circuit sont par exemple du type LM 308. Le circuit de la Fig.6 est réglé au moyen du potentiomètre 38 pour réguler le débit de la pompe 1 en agissant sur l'alimentation de son moteur électrique d'entraînement. Le signal de sortie du débitmètre indiquant le débit instantané de la pompe matérialisé par le signal $\Delta p$ est appliqué par l'intermédiaire du circuit de lissage 30 à l'entrée de l'amplificateur opérationnel 36 qui reçoit également le signal de référence correspondant au débit à maintenir que lui applique le potentiomètre 38 par l'intermédiaire de la résistance 39. Le signal résultant de la sommation de ces deux signaux, est appliqué aux entrées des amplificateurs 44 et 45 qui délivrent à leurs sorties, un signal assurant la conduction du transistor 49 et de ce fait du transistor 50, et par conséquent, l'alimentation plus ou moins importante du moteur d'entraînement de la pompe 1 en fonction du degré de conduction de l'amplificateur 50 lorsque le débit de la pompe doit être augmenté ou diminué.

On obtient ainsi à la sortie de la pompe un débit constant qui rend les mesures de l'analyseur à infrarouges cohérentes au cours de toute la durée de la combustion d'un échantillon dont il y a lieu de déterminer la teneur en CO2 et par conséquent en carbone.

Compte tenu que le détecteur à infra-rouges mesure la pression partielle de $CO_2$ dans $O_2$, il y a lieu, pour augmenter la précision de la mesure, de compenser le signal issu du détecteur par un signal en relation avec la variation de la pression atmosphérique.

En conséquence, l'analyseur suivant l'invention comporte un circuit 5 de compensation de pression atmosphérique disposé entre l'analyseur à infrarouges 4 et le circuit de traitement du signal représenté à la Fig.3.

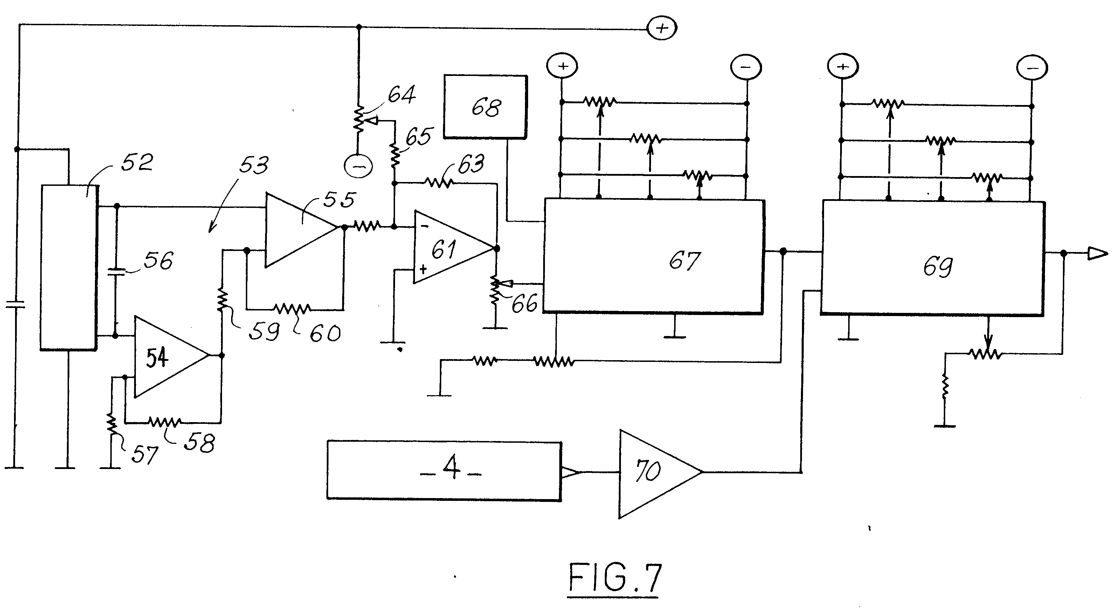

Ce circuit de compensation de pression atmosphérique représenté plus en détail à la Fig.7 comporte essentiellement un capteur de pression atmosphérique 52 à la sortie duquel est connecté un circuit de conditionnement du signal de pression atmosphérique 53 constitué de deux amplificateurs opérationnels 54 et 55, par exemple du type LM 308 dont les bornes non inverseuses sont respectivement connectées aux deux bornes de sortie du capteur de pression 52 en parallèle sur un condensateur 56. La borne inverseuse de l'amplificateur 54 est connectée d'une part à la masse par l'intermédiaire d'une résistance 57 et d'autre part, à la sortie de cet amplificateur par une autre résistance 58. La sortie de l'amplificateur 54 est connectée à l'entrée inverseuse de l'amplificateur 55 par l'intermédiaire d'une résistance 59 tandis qu'une résistance 60 est connectée en parallèle entre l'entrée inverseuse de l'amplificateur 55 et sa sortie. Cette dernière est reliée à l'entrée inverseuse d'un amplificateur opérationnel 61 par l'intermédiaire d'une résistance 62; l'entrée non-inverseuse de cet amplificateur est connectée à la masse, son entrée inverseuse est en outre connectée à sa sortie par l'intermédiaire d'une résistance de réaction 63 connectée à la tension d'alimentation par l'intermédiaire d'un potentiomètre 64 dont le curseur est relié à l'entrée non inverseuse de l'amplificateur 61 par l'intermédiaire d'une résistance 65.

La sortie de l'amplificateur 61 est connectée par l'intermédiaire d'un potentiomètre 66 à l'entrée d'un premier diviseur 67 destiné à engendrer le rapport P/PO et qui reçoit sur une autre de ses entrées, un signal de référence correspondant à la pression PO engendrée par un circuit en pont 68.

La sortie du diviseur 67 est connectée à une entrée d'un autre diviseur 69 relié par une autre de ses entrées à l'analyseur à infrarouges 4 (Fig.1) par l'intermédiaire d'un amplificateur 70. La fonction de ce second diviseur 69 est de générer un signal correspondant au produit S x PO / P du signal de sortie de l'analyseur à infrarouges par le rapport PO/P inverse du rapport P/PO généré par le diviseur 67. Ainsi, à la sortie du diviseur 69, apparaît un signal Sc correspondant au signal de mesure de l'analyseur à infrarouges compensé en fonction des variations de la pression atmosphérique.

Dans le présent exemple, la fonction de transfert du diviseur 67 est Vc = 10 VP / VPO. Si l'on admet que VPO = - 10 V,
VP = 7,6 V pour 760 mm de Mg, alors la fonction de transfert du diviseur 69 est VF = 10 x Va / Vc, Va = 760 mV pour PE, ce qui donne VZinB = - 7,6 V pour PE.

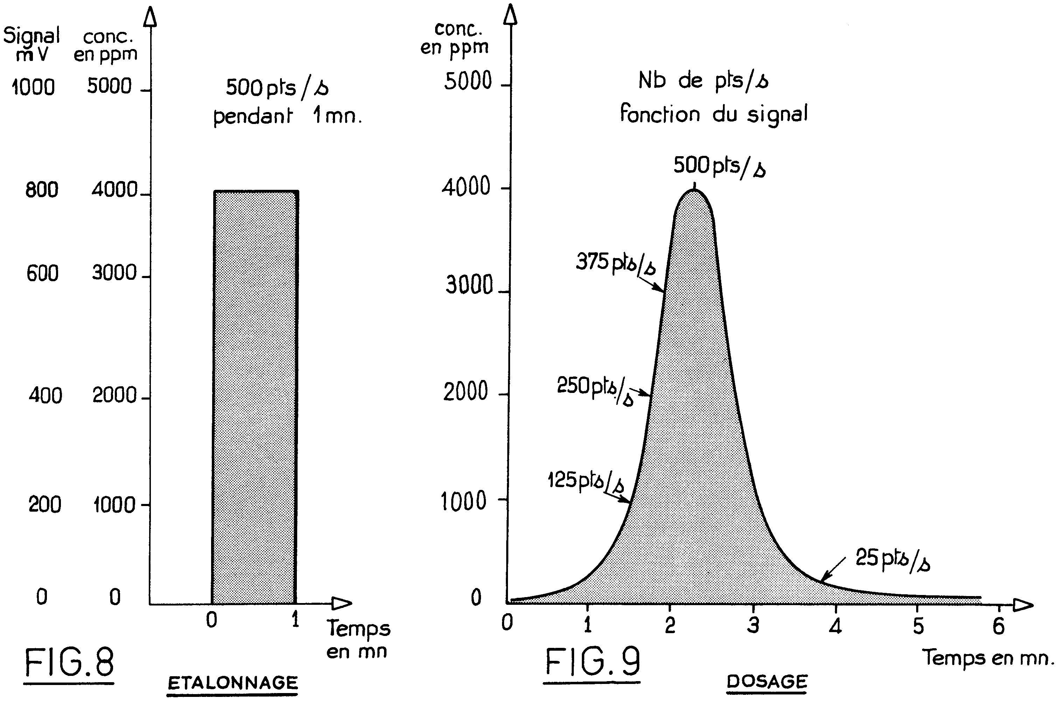

L'étalonnage de l'analyseur suivant l'invention va maintenant être décrit en référence aux Fig.8 et 9.

On commence par étalonner l'analyseur à infrarouges 4. A cet effet, on injecte à l'entrée du four (non représenté), de l'oxygène pur qui sert ultérieurement pour la mesure. On attend que le dispositif d'affichage 12 de l'analyseur à infrarouges se stabilise dans une position d'équilibre. La circulation du gaz peut être assurée pendant la période de chauffe pendant laquelle on ajuste le zéro du dispositif d'affichage.

On injecte ensuite un gaz étalon et l'on attend que la lecture de l'afficheur se stabilise. On ajuste alors l'afficheur à la concentration en CO2 du gaz étalon.

Pour assurer l'étalonnage de l'intégrateur du circuit de traitement du signal de sortie de l'analyseur à infrarouges, on fait passer dans l'analyseur du gaz étalon et l'on obtient à la sortie du circuit de traitement du signal de la Fig.3, une impulsion rectangulaire d'étalonnage stable telle que représentée à la Fig.8.

L'étalonnage dure une minute.

Cet afficheur indique la quantité de CO2 ayant traversé la cellule 8 de l'analyseur à infrarouges de la Fig.2 pendant une minute. Il peut alors être ajusté pour une lecture directe de la quantité de carbone correspondante.

Dans les conditions prévues dans le présent exemple, la quantité de gaz d'étalon est de 4080 ppm de CO2 dans O2 en moles. Le débit est de 500 ml/mn pour un échantillon de 150 x 300 mm. La quantité de carbone superficielle est $C_{surface} = 12,14$ mg/m$^2$.

$C_{acier} = 109, 1.10^{-3}$ % pour une PE de 1g. On ne modifie alors plus les réglages précédents.

L'analyse des échantillons est alors réalisée de la manière suivante.

L'analyse est déclenchée.

On suit sur l'enregistreur 19 du circuit de traitement de signal représenté à la Fig.3, la valeur instantanée de la quantité de carbone contenue dans le gaz analysé. Lorsqu'on observe sur la courbe que la concentration en CO2 n'évolue presque plus au voisinage de zéro, le dosage est terminé et on arrête la mesure.

Pendant l'analyse, l'afficheur 18 indique la quantité de carbone qui est passée à travers la cellule 8 de l'analyseur infrarouges.

Un exemple d'une mesure de la quantité de carbone contenue dans le gaz de combustion d'un échantillon dans un four est représenté à la Fig.9 sur laquelle on voit l'évolution du nombre de points par seconde, au cours d'une mesure complète qui dure dans l'exemple considéré environ 6 mn. La comparaison de la surface délimitée par la courbe de la Fig.9 à la courbe d'étalonnage de la Fig.8 donne une mesure de la quantité de carbone ayant traversé la cellule de l'analyseur à infrarouges pendant une période de mesure complète.

**Revendications**

1. Dispositif de mesure de la quantité d'un constituant contenu dans un échantillon de produit, comprenant associés à un four pour brûler l'échantillon de produit, des moyens (1) pour mettre l'atmosphère du four en circulation à travers un débitmètre (3) et un analyseur à infrarouges (4) qui délivre à sa sortie un signal de mesure instantanée de la quantité de gaz contenant le constituant, contenue dans l'atmosphère du four, caractérisé en ce qu'il comporte en outre des moyens d'intégration de la mesure instantanée de la quantité de gaz contenant le constituant, comprenant un convertisseur tension-fréquence (15) de conversion de la tension instantanée de sortie de l'analyseur en une fréquence proportionnelle à la tension instantanée et un compteur binaire (16) des pics des signaux de fréquence émis par le convertisseur et dont le compte représente la quantité de gaz contenant le constituant ayant traversé l'analyseur à infrarouges.

2. Dispositif suivant la revendication 1, caractérisé en ce qu'il comporte en outre un convertisseur numérique-analogique (17) des signaux de sortie du compteur binaire (16) destiné à délivrer à la fin d'une période de mesure un signal proportionnel à la surface délimitée par la courbe d'évolution de la teneur en gaz à mesurer et des moyens (18) d'affichage de la quantité de produit à mesurer contenue dans l'échantillon.

3. Dispositif suivant l'une des revendications 1 et 2, caractérisé en ce que le compteur binaire est un compteur à seize bits et comporte un diviseur par seize (24) connecté à la sortie du convertisseur tension-fréquence (15) par l'intermédiaire d'une porte (23) également connectée à un circuit de validation (24) et deux compteurs (25,26) dont les sorties forment un mot de seize bits représentant la quantité de constituant ayant traversé l'analyseur à infrarouges au cours d'une mesure.

4. Dispositif suivant l'une des revendications 1 à 3, caractérisé en ce que les moyens de mise en circulation de l'atmosphère du four étant constitués par une pompe (1) entraînée par un moteur électrique, il comporte en outre un circuit (6) de régulation du débit de la pompe à une valeur constante, connecté entre le débitmètre (3) et le moteur électrique d'entraînement de la pompe (1).

5. Dispositif suivant la revendication 4, caractérisé en ce que le circuit de régulation (6) comporte un sommateur (36) du signal ($\Delta p$) de sortie du débitmètre (3) et d'un signal de référence délivré par un circuit (38,39) de réglage de référence de débit, un amplificateur intégral (44) et un amplificateur proportionnel (45) connectés au circuit sommateur (36) et dont les signaux de sortie sont combinés dans un potentiomètre (48) pour commander le degré de conduction d'un transistor (50) dont le trajet émetteur-collecteur est connecté dans le circuit d'alimentation du moteur électrique d'alimentation de la pompe (1).

6. Dispositif suivant l'une des revendications 1 à 5, caractérisé en ce qu'il comporte en outre un circuit (5) de compensation des variations de la pression atmosphérique interposé entre la sortie de l'analyseur à infrarouges (5) et l'entrée du convertisseur tension-fréquence (15) desdits moyens d'intégration.

7. Dispositif suivant la revendication 6, caractérisé en ce que le circuit de compensation des variations de la pression atmosphérique comporte un capteur (52) de la pression atmosphérique P et des diviseurs (67,69) destinés à délivrer à partir du signal de sortie du capteur (52) correspondant à la pression atmosphérique P et d'un signal correspondant à la pression Po délivré par un circuit du pont (68) et du signal de sortie S de l'analyseur à infrarouges (4), un signal correspondant à la valeur Sc = S x Po/P du signal de sortie de l'analyseur à infrarouges compensé en fonction de la variation de la pression atmosphérique.

FIG.1

FIG.2

FIG.3

FIG.4

FIG.5

FIG.6

10

FIG.7

EP 0 512 891 A1

FIG.8

ETALONNAGE

FIG.9

DOSAGE

EP 0 512 891 A1

EP 0 512 891 A1

**Office européen des brevets**

# RAPPORT DE RECHERCHE EUROPEENNE

Numero de la demande

EP 92 40 1210

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | US-A-4 845 040 (K.S. MOON ET AL.)<br>* revendication 8 *<br>--- | 1 | G01N33/20<br>G01N21/35<br>G01N31/12 |
| A | STAHL UND EISEN<br>no. 3, Février 1984, DUSSELDORF, DEUTSCHLAND<br>pages 141 - 144;<br>H. LEMM ET AL.: 'BESTIMMUNG DES OBERFLACHENKOHLENSTOFFS AUF STAHL DURCH DIREKTE VERBRENNUNG IM SAUERSTOFFPLASMA'<br>* page 143, colonne de gauche *<br>--- | 1 | |
| A | US-A-4 332 770 (KOZO ISHIDA ET AL.)<br>* revendications 1,2 *<br>--- | 1 | |
| A | FR-A-2 556 838 (NIPPON STEEL CORPORATION)<br>* revendication 1 *<br>--- | 1 | |
| A | US-A-3 181 343 (J.D. FILLON)<br>* revendications 20,23-25 *<br><br>----- | 1 | |

DOMAINES TECHNIQUES RECHERCHES (Int. Cl.5)

G01N

Le présent rapport a été établi pour toutes les revendications

| Lieu de la recherche | Date d'achèvement de la recherche | Examinateur |
|---|---|---|
| LA HAYE | 20 AOUT 1992 | VAN DEN BULCKE E.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

13